# EUROPEAN PATENT APPLICATION

(11) **EP 2 505 122 A1**
(43) Date of publication of application: **03.10.2012**
(21) Application number: 12162125.4
(22) Date of filing: 29.03.2012
(51) Int. Cl.: A61B 1/06, A61B 1/00

(54) **Endoscope system and calibration method**

(30) Priority: 01.04.2011 JP 2011081757; 15.03.2012 JP 2012058199
(71) Applicant: Fujifilm Corporation, Minato-ku Tokyo (JP)
(72) Inventor: Minetoma, Yasuhiro, Kanagawa (JP); Kaku, Toshihiko, Kanagawa (JP)
(74) Representative: Klunker . Schmitt-Nilson . Hirsch

(57) **Abstract**

An endoscope system includes an illumination apparatus (14, 104) for applying imaging light to body tissue in a tube of a body cavity. An electronic endoscope (12) images the body tissue illuminated with the imaging light, and outputs a blue image signal (S'B) of blue and a green image signal (S'G) of green. A signal processing device detects specific body tissue (such as blood vessels) in the body tissue according to the blue and green image signals. A calibration device (52, 60) refers to a reference ratio (SB/SG) predetermined according to a reflectivity of the body tissue in relation to the blue and green, and calibrates the blue and green image signals to set a ratio between the blue and green image signals equal to the reference ratio.

## Description

### BACKGROUND OF THE INVENTION

### 1. Field of the Invention

The present invention relates to an endoscope system and a calibration method. More particularly, the present invention relates to an endoscope system and a calibration method, in which special light mode imaging is carried out with special light, and body tissue can be imaged in the special light mode imaging stably without influence of finely different characteristics of system components such as an endoscope and an illumination apparatus.

### 2. Description Related to the Prior Art

An electronic endoscope is a medical instrument used widely for diagnosis and treatment. Normal light or white light is applied to body tissue of a body cavity of a patient. However, it may be rather difficult to view the body tissue only with the normal light. There is a type of the endoscope in which special light is applied to the body tissue in a predetermined wavelength range of a narrow band. In special light mode imaging by use of the special light, contrast of a target portion of the body tissue to its remaining portion can be high, the target portion absorbing the special light at a high ratio. The target portion can be enhanced over normal mode imaging by use of the normal light.

An endoscope system includes the endoscope, a processing apparatus and an illumination apparatus. The endoscope is entered in a body cavity of a patient. The processing apparatus creates image data according to an image signal, and processes the image signal for image processing. The illumination apparatus supplies the endoscope with light for illumination. Such system components or apparatuses in the endoscope system are combined together, and if required, can be exchanged discretely. For examples, a plurality of types of the endoscope are prepared for different body parts as objects of interest, with differences in a diameter of an elongated tube and the like. One endoscope is selected for use with one of the body parts. Also, the illumination apparatus can be exchanged for the special light mode imaging or according to presence or lack of a light source with a wavelength required for the special light mode imaging.

There is a type difference between types of the system components of the endoscope system because of differences in characteristics of a CCD image sensor (detection unit), the light source and the like. Also, there is an individual difference (specificity) of the CCD or the light source in relation to characteristics even though the system components have been manufactured in an equal form. The processing apparatus is conditioned constantly for signal processing and image processing. Thus, the type differences and the individual difference of the system components will cause harmful influence to diagnosis, because an image is obtained with color balance different from an object of the image, or its image quality may be lower. There is a technical requirement for minimizing influence of the individual difference of the system components in the imaging. If any one of the system components is exchanged in the endoscope system, imaging should be conditioned equally.

In the endoscope, the image signal from the CCD is transmitted to the processing apparatus by a signal cable such as a universal cable. If a length of the signal cable changes due to a change in a condition for connection, a waveform of the image signal is changed to lower the quality of an image. In view of this problem, JP-A 2000-342533 discloses the endoscope system in which information of the length of the signal cable is stored in the endoscope, and the waveform of the image signal is corrected in a form of a predetermined waveform according to the length of the signal cable, so as to obtain an image with high image quality irrespective of the individual difference of the endoscope.

In the use of the special light mode imaging in the endoscope system, a target portion of the body tissue is enhanced for imaging by utilizing a difference in the absorption according to the wavelength. Should the color balance of an image be changed finely by the individual difference of the endoscope or the illumination apparatus, the contrast of the body tissue abruptly drops, so that imaging will be very difficult without successful enhancement. Reduction of the individual difference of the endoscope or the illumination apparatus is important particularly in the endoscope system for the special light mode imaging.

In the conventional endoscope system, the color balance of an image is adjusted by adjusting the white balance of the image signal. However, it is likely that the body tissue of interest is not enhanced sufficiently in the special light mode imaging only the control of the white color. It is still difficult to image the body tissue in the special light mode imaging additionally due to the individual difference of the system components, because of insufficient definition and the like.

The endoscope system of JP-A 2000-342533 stores information of characteristics of the CCD, length of the signal cable as the individual difference of the endoscope to correct the waveform of the image signal. However, only effect equal to that of white balance control is obtained. It is impossible to reduce influence of the individual difference of the endoscope and the illumination apparatus in the special light mode imaging.

### SUMMARY OF THE INVENTION

In view of the foregoing problems, an object of the present invention is to provide an endoscope system and a calibration method, in which special light mode imaging is carried out with special light, and body tissue can be imaged in the special light mode imaging stably without influence of finely different characteristics of system components such as an endoscope and an illumination apparatus.

In order to achieve the above and other objects and advantages of this invention, an electronic endoscope system for imaging body tissue in a tube of a body cavity is provided, and includes an illumination apparatus for applying imaging light to the body tissue. There is a detection unit for imaging the body tissue illuminated with the imaging light, and for outputting a first color image signal of a color of a first wavelength range and a second color image signal of a color of a second wavelength range. A calibration device refers to a reference ratio predetermined according to a reflectivity of the body tissue in relation to the first and second wavelength ranges, and calibrates the first and second color image signals to set a ratio between the first and second color image signals equal to the reference ratio.

Furthermore, a signal processing device detects specific body tissue in the body tissue according to the first and second color image signals.

The calibration device carries out calibration when at least one of the illumination apparatus and the detection unit is exchanged.

The reference ratio is determined according to the first and second color image signals obtained by imaging with a reference illumination apparatus and a reference detection unit.

The reference ratio is determined by a reference image signal obtained by imaging a reference chart, and the reference chart has a spectrum of reflection of the body tissue.

The first wavelength range is equal to or more than 400 nm and equal to or less than 500 nm, and the second wavelength range is equal to or more than 500 nm and equal to or less than 600 nm.

Furthermore, a memory stores a color calibration table for associating a second pair of the first and second color image signals with a first pair of the first and second color image signals, the second pair being obtained by imaging the reference chart by use of the illumination apparatus for use and the endoscope for use, the first pair being obtained by imaging the reference chart by use of a reference illumination apparatus and a reference endoscope. The calibration device carries out calibration according to the color calibration table.

Furthermore, a display control unit creates a display image by use of the first and second color image signals.

The display control unit creates a special image for the display image by allocating a signal level of the first color image signal to blue and green pixels, and allocating a signal level of the second color image signal to red pixels.

The signal processing device evaluates the ratio of the first and second color image signals by pixels, and extracts the specific body tissue from an image of the body tissue according to evaluation of the ratio.

The imaging light is special light having a first peak wavelength corresponding to blue, and a second peak wavelength shorter than the first peak wavelength and corresponding to blue violet.

The detection unit includes a color image sensor. The illumination apparatus includes a blue laser for generating a blue laser beam. A blue violet laser generates a blue violet laser beam. Phosphor is disposed at a distal end of an endoscope having the detection unit, for generating fluorescent light from green to yellow upon emission of the blue laser beam and the blue violet laser beam, wherein white light is generated in combination of the fluorescent light and blue light upon passage through the phosphor, and becomes the imaging light to the body tissue.

The imaging light contains a white light component, and the endoscope includes a full-color image sensor.

In a preferred embodiment, the imaging light contains blue narrow band light and green narrow band light applied to the body tissue sequentially. The endoscope includes a monochromatic image sensor for outputting the first and second color image signals serially.

Also, a calibration method of calibrating a first endoscope system by use of a reference endoscope system is provided. Each of the reference endoscope system and the first endoscope system includes an illumination apparatus for applying imaging light to body tissue in a tube of a body cavity, and an electronic endoscope for imaging the body tissue illuminated with the imaging light, and for outputting a first color image signal of a color of a first wavelength range and a second color image signal of a color of a second wavelength range. The calibration method includes a step of imaging a reference chart by use of the reference endoscope system to obtain a first pair of the first and second color image signals, the reference chart having a color pattern according to a surface color of the body tissue. A reference ratio between the first and second color image signals of the first pair is determined. The reference chart is imaged by use of the first endoscope system to obtain a second pair of the first and second color image signals. A ratio between the first and second color image signals of the second pair is determined. The second pair of the first and second color image signals is calibrated by setting the ratio equal to the reference ratio, to control a color balance of an image output by the first endoscope system.

The illumination apparatus is used commonly between the reference endoscope system and the first endoscope system.

Also, an endoscope system includes an illumination apparatus for applying imaging light to body tissue in a tube of a body cavity. An electronic endoscope images the body tissue illuminated with the imaging light, and outputs a first color image signal of a color of a first wavelength range and a second color image signal of a color of a second wavelength range. A signal processing device detects specific body tissue in the body tissue according to the first and second color image signals. A calibration device refers to a reference ratio predetermined according to a reflectivity of the body tissue in relation to the first and second wavelength ranges, and calibrates the first and second color image signals to set a ratio between the first and second color image signals equal to the reference ratio.

Consequently, body tissue can be imaged in the special light mode imaging stably without influence of finely different characteristics of system components such as an endoscope and an illumination apparatus, because such differences are compensated for by means of the calibration of color image signals according to the reference ratio.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating an endoscope system;
Fig. 2 is a block diagram schematically illustrating the endoscope system;
Fig. 3 is an explanatory view illustrating creation of mode image data by calibration of an image signal;
Fig. 4 is a flow chart illustrating creation of a color calibration table;
Fig. 5 is a table illustrating a color calibration table;
Fig. 6 is a graph illustrating spectra of light reflected by body tissue;
Fig. 7 is a graph illustrating spectra of source light;
Fig. 8 is a graph illustrating characteristics of a color filter;
Fig. 9 is a graph illustrating an image signal obtained by imaging a calibration chart;
Fig. 10 is a graph illustrating calibration of an image signal;
Fig. 11 is a graph illustrating an image signal obtained in white balance control;
Fig. 12 is a graph illustrating a relationship between a tissue depth and a ratio B/G;
Fig. 13 is a block diagram schematically illustrating an endoscope system of a frame sequential type;
Fig. 14 is a plan illustrating a filter wheel;
Fig. 15 is a graph illustrating spectra of narrow band light;
Fig. 16 is an explanatory view illustrating creation of mode image data in the endoscope system of the frame sequential type.

### DETAILED DESCRIPTION OF THE PREFERRED EMBODIMENT(S) OF THE PRESENT INVENTION

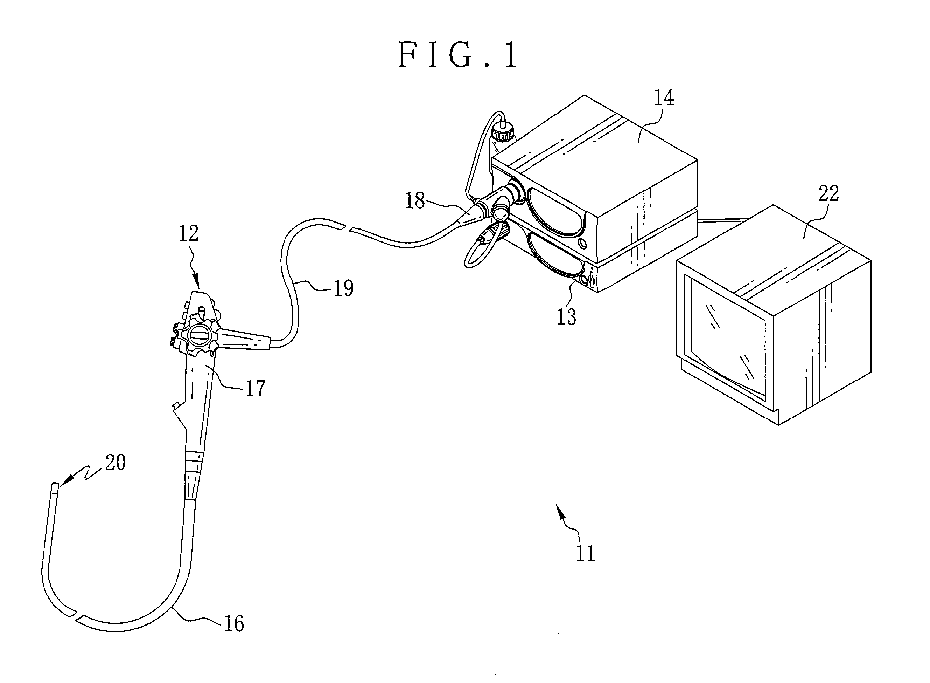

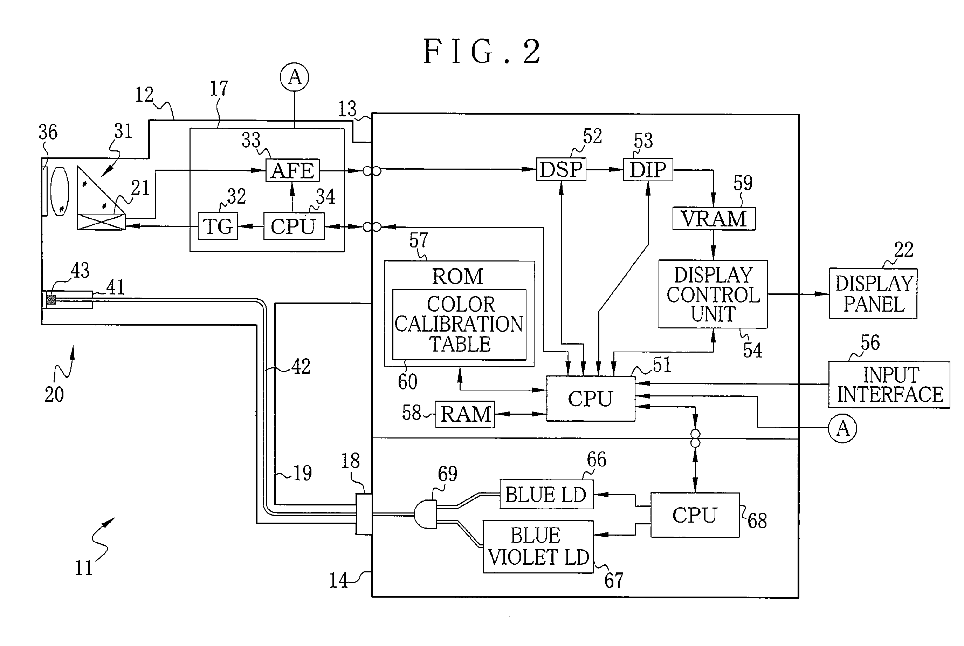

In Fig. 1, an endoscope system 11 includes an electronic endoscope 12, a processing apparatus 13 and an illumination apparatus 14. Types of the endoscope 12 and the illumination apparatus 14 are changeable for the purpose of use, namely for a body part of a patient. The endoscope 12 includes a section of an elongated tube 16 or guide tube, a handle device 17, a connector plug 18 and a universal cable 19. The elongated tube 16 is flexibly entered in a body cavity. The handle device 17 is disposed at a proximal end of the elongated tube 16. The connector plug 18 is connected to the processing apparatus 13 and the illumination apparatus 14. The universal cable 19 extends between the handle device 17 and the connector plug 18 for connection. A head assembly 20 is disposed at a distal end of the elongated tube 16. A CCD image sensor 21 (detection unit) is incorporated in the head assembly 20 for imaging of body tissue of the body cavity. See Fig. 2.

The handle device 17 includes steering wheels, a fluid supply button, a release button, a zoom button, a mode button and the like. The steering wheels are rotatable to steer the head assembly 20 up and down and to the right and left. The fluid supply button supplies air and water for ejection through an end nozzle of the elongated tube 16. The release button records an object image in a form of a still image. The zoom button designates enlargement and reduction of the object image. A monitor display panel 22 displays the object image and is viewed for operating the zoom button. The mode button changes over normal mode imaging and special light mode imaging.

The processing apparatus 13 is connected electrically with the illumination apparatus 14, and also controls the various elements of the endoscope system 11. The processing apparatus 13 supplies the endoscope 12 with power through a cable extending along the universal cable 19 and through the elongated tube 16, and controls the CCD 21. The processing apparatus 13 receives an image signal output by the CCD 21 through the cable, processes the image signal in image processing of various functions, and creates image data of an object image. The image of the image data from the processing apparatus 13 is displayed on the monitor display panel 22 which is connected to the processing apparatus 13 by a cable.

In Fig. 2, the head assembly 20 has an objective lens system 31 and a lighting unit 41 with a lens in addition to the CCD 21. Various elements are incorporated in the handle device 17, including a timing generator 32 (TG), an analog signal processor 33 (AFE) and a CPU 34.

The objective lens system 31 includes a lens, prism and the like. There is an imaging window 36 through which object light is incident upon the objective lens system 31, which focuses the object light on the CCD 21.

The CCD 21 or a full-color image sensor photoelectrically converts image data of an object image focused by the objective lens system 31 on an imaging surface for respective pixels, and stores signal charge according to an amount of incident light. The CCD 21 outputs an image signal according to the stored signal charge from the pixels. Also, a color filter device is formed on the CCD 21, and is constituted by plural color filter segments for the pixels. An example of color filter device of the CCD 21 is a color filter of a Bayer filter array of R, G and B.

The timing generator 32 inputs a clock signal to the CCD 21. In response to this, the CCD 21 operates sequentially for storing signal charge and reading the signal charge. The clock signal from the timing generator 32 is controlled by the CPU 34.

The analog signal processor 33 includes a correlated double sampling circuit (CDS), auto gain control circuit (AGC), A/D converter and the like. The analog signal processor 33 is supplied with an image signal of an analog form, and removes electric noise from the image signal. The analog signal processor 33 corrects the image signal for the gain correction, and converts the same into a digital signal. A digital signal processing device 52 (DSP) is supplied with the digital signal. The CDS operates according to the correlated double sampling, removes the noise derived from operation of the CCD 21, and obtains the image signal. The AGC amplifies the image signal from the CDS. The A/D converter converts the image signal from the AGC into a digital image signal with bits of a predetermined number, and sends the digital image signal to the digital signal processing device 52. The analog signal processor 33 is controlled by the CPU 34. A CPU 51 is incorporated in the processing apparatus 13. The CPU 34 adjusts a gain of the image signal in the AGC according to a control signal from the CPU 51.

The lighting unit 41 applies light to body tissue of a body cavity. Normal light and special light is emitted by the lighting unit 41 for illumination. Note that the normal light and special light is emitted simultaneously by the lighting unit 41 to be described later.

The lighting unit 41 includes phosphor 43. A light guide device 42 is constituted by fiber optics, and guides blue laser light or blue violet laser light from the illumination apparatus 14. The phosphor 43 excites to emit light from green to yellow by partially absorbing blue laser light or blue violet laser light. Examples of the phosphor 43 are YAG phosphor, BAM phosphor (BaMgAl₁₀O₁₇) and the like. The blue light and blue violet light guided into the lighting unit 41 is partially absorbed by the phosphor 43, which emits fluorescent light from green to yellow. Part of the light passes through the phosphor 43. Thus, the lighting unit 41 applies composite light to body tissue of a body cavity, the composite light being normal light (pseudo white light) after combination of the fluorescent light from green to yellow from the phosphor 43, and the blue light transmitted by the phosphor 43. At the same time, blue light and blue violet light from the phosphor 43 operates also as special light.

Efficiency in light emission of excitation of the phosphor 43 is different between the blue laser light and blue violet laser light. An amount of fluorescent light generated according to the blue laser light is more than that generated according to the blue violet laser light under a condition of an equal light amount of incidence. Also, the blue laser light is diffused by the phosphor 43 upon transmission. Normal light emitted by the lighting unit 41 is regular in a field of view of the endoscope 12.

The processing apparatus 13 has the CPU 51 and the digital signal processing device 52, and also includes a digital image processor 53 (DIP), a display control unit 54 and an input interface 56.

The CPU 51 is connected to various elements of the processing apparatus 13 by a data bus, an address bus and lines (not shown), and controls the processing apparatus 13. A ROM 57 stores programs, graphic data and control data. The programs include an operation system (OS), application programs and the like for controlling the processing apparatus 13. An example of the control data is a color calibration table 60 (for a calibration device). A RAM 58 is a working memory. The CPU 51 reads a program and data from the ROM 57 as required by use of the RAM 58, and processes the program sequentially. Also, the CPU 51 retrieves information through the input interface 56 or the network such as LAN, and writes the information to the RAM 58, the information including a patient' s name, operator' s name, examination date and time and other alphanumeric information specific to one case of the examination or imaging.

The digital signal processing device 52 receives the image signal through the analog signal processor 33 through the CCD 21, and processes the signal according to signal processing, for example, color separation, color interpolation, gain correction, white balance control, gamma correction and the like. The white balance control is carried out for normal mode imaging. Color adjustment is carried out for special light mode imaging in other embodiment modes to be described later.

In the normal mode imaging, the digital signal processing device 52 creates normal image data in which a blue signal (blue image signal) from blue pixels of the CCD 21 is allocated to blue pixels, a green signal (green image signal) from green pixels of the CCD 21 is allocated to green pixels, and a red signal (red image signal) from red pixels of the CCD 21 is allocated to red pixels.

In the special light mode imaging, the digital signal processing device 52 creates mode image data (special light mode image data or color enhanced image data) in which the blue signal is allocated to the blue pixels and green pixels, and the green signal is allocated to the red pixels. The red signal is abandoned. Also, the digital signal processing device 52 reads the color calibration table 60 from the ROM 57, and processes the image signal after calibration of signal levels of blue, green and red image signals according to the color calibration table 60 for creation of mode image data.

The color calibration table 60 is a conversion table of data for calibrating signal levels of image signals from the CCD 21 by each of the colors, and is previously obtained according to characteristics of the endoscope 12 and the illumination apparatus 14. The data in the color calibration table 60 are so formed that a signal level of an image signal comes up to represent a predetermined color balance. A ratio B/G of the blue signal to the green signal after the calibration is set equal to a predetermined reference ratio among the image signals calibrated with the color calibration table 60. Even when an individual difference (specificity) occurs in the endoscope 12 or the illumination apparatus 14 with characteristics different from reference characteristics, mode image data created from the calibrated image signals is in such a form that the ratio B/G is equal to the reference ratio. The reference ratio for B/G is predetermined so as to obtain image data with high contrast of blood vessels by use of the reference endoscope 12 and the reference illumination apparatus 14.

The image data created by the digital signal processing device 52 is stored in a working memory of the digital image processor 53. Also, the digital signal processing device 52 creates control data for ALC and inputs the control data to the CPU 51, the control data including an average luminance value of pixels of image data and the like for the ALC or automatic control of lighting.

The digital image processor 53 processes the image data from the digital signal processing device 52 in image processing of various functions, such as electronic zooming and enhancement. There is a VRAM 59 in which the processed image data from the digital image processor 53 is stored in a temporary manner. Then the processed image data is sent to the display control unit 54. The enhancement in the image processing in the digital image processor 53 is frequency enhancement, and used when required. The digital image processor 53 increases contrast of an image to be enhanced by increasing pixel values in an image of a predetermined frequency range, for various states of body tissue for enhancement, such as surface blood vessels, deep blood vessels, and the like.

The display control unit 54 retrieves an object image from the VRAM 59, and receives graphic data through the CPU 51 from the ROM 57 and the RAM 58. Examples of the graphic data include data of a display mask, alphanumeric information such as a patient's name, operator's name, examination date and time, data of graphic user interface (GUI), and the like. The display mask is used to display only an active pixel area where an object is located within the object image. The display control unit 54 superimposes graphic data on the object image, and converts the image into a video signal (component signal, composite signal and the like) according to a display format of the monitor display panel 22, and outputs the video signal to the monitor display panel 22. Thus, the monitor display panel 22 displays the object image.

The input interface 56 is disposed on a housing of the processing apparatus 13. Examples of the input interface 56 include any of an input panel, mouse, keyboard and other well-known input devices. The CPU 51 receives a control signal from the handle device 17 of the endoscope 12 or the input interface 56, and causes various elements of the endoscope system 11 to operate.

The processing apparatus 13 also includes a compressor, a media interface and a network interface. The compressor compresses image data according to a predetermined compression format, for example JPEG. The media interface writes the compressed image data to a removable storage medium in response to depression of the release button. The network interface transfers various data to and from the LAN or other network. Those are connected to the CPU 51 by a data bus or the like.

The illumination apparatus 14 includes a blue laser diode 66 (LD) and a blue violet laser diode 67 (LD) for lighting.

The blue LD 66 emits blue laser light with a center wavelength of 445 nm. The blue laser light is guided through the connector plug 18 and the light guide device 42 to the lighting unit 41. The phosphor 43 converts the blue laser light into normal light of pseudo white color, which is applied to a body cavity. Also, the blue laser light is diffused by transmission through the phosphor 43, and becomes blue light which is applied to the body cavity. The blue light is stronger than fluorescent light from the phosphor 43 upon excitation, and is used as special light highly absorbable in blood in surface blood vessels.

The blue violet LD 67 emits blue violet laser light with a center wavelength of 405 nm. An optical coupler 69 combines the blue violet laser light with the blue laser light. The blue violet laser light is directed through the connector plug 18 and the light guide device 42 to the lighting unit 41 in a manner similar to the blue laser light. The blue violet laser light is incident on the phosphor 43, and becomes normal light of a pseudo white color before application to a body cavity. A light amount of this light is generally smaller than that of light formed from the blue laser light. Blue violet light, output by diffusion of the blue violet laser light through the phosphor 43, is applied as special light in a manner similar to blue light.

A CPU 68 controls a sequence of light emission, a light amount and the like of the blue and blue violet LDs 66 and 67. For example, only the blue LD 66 is turned on for the normal mode imaging. The blue and blue violet LDs 66 and 67 are turned on for the special light mode imaging. Also, the CPU 68 controls the light amount of the blue and blue violet LDs 66 and 67 automatically at real time in an optimized manner for imaging according to the ALC control data received from the CPU 51 of the processing apparatus 13.

For the endoscope system 11, the endoscope 12 and the illumination apparatus 14 are selected from plural types suitably according to a body part to be imaged. The endoscope 12 and the illumination apparatus 14 after the selection are connected to the processing apparatus 13 for use. The blue and blue violet LDs 66 and 67 are turned on at the same time irrespective of the normal mode imaging and special light mode imaging. The lighting unit 41 emits white light and special light (blue and blue violet) simultaneously to irradiate an object of interest. Note that light amounts of the blue and blue violet LDs 66 and 67 and a ratio between their light amounts are adjusted according to a selected one of the normal mode imaging and special light mode imaging, enhancement of a selected one of surface blood vessels and deep blood vessels, and the like.

During the normal mode imaging, the endoscope system 11 creates normal image data by using the blue signal for the blue pixels, using the green signal for the green pixels, and using the red signal for the red pixels according to outputs from the CCD 21. The normal image data is processed in image processing of various functions by the digital image processor 53, and combined with graphic data by the display control unit 54 in superimposition, so that the monitor display panel 22 displays the image.

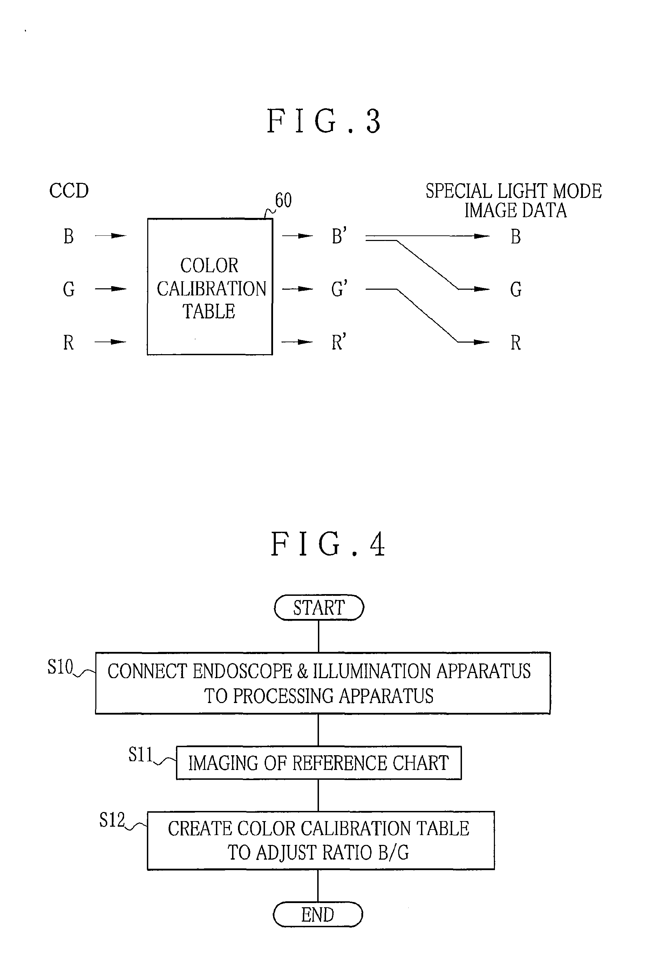

In Fig. 3, the endoscope system 11 calibrates the color image signals to obtain the B' , G' and R' signals according to the color calibration table 60 for the special light mode imaging, the color image signals being generated by the CCD 21 for the primary colors. Then mode image data (special light mode image data or color enhanced image data) is created by using a B' signal for blue and green pixels, and by using a G' signal for red pixels, the B' signal being a blue image signal after the calibration, the G' signal being a green image signal after the calibration. In an image of the mode image data obtained in this manner, blood vessels are enhanced over an image of the normal image data. This is because the peak of light absorption of hemoglobin in blood is included in each of wavelength ranges of blue and green light. Contrast of blood vessels according to the blue and green signals increases in a corresponding manner. The mode image data is processed by the digital image processor 53 in the image processing of functions designated by the control signal, before the display control unit 54 combines the graphic data to the mode image data, of which a composite image is displayed on the monitor display panel 22.

There are deviations in the sensitivity of pixels of the CCD 21, characteristic of the color filter, and the like in the endoscope 12. There are deviations in the center wavelengths of the blue and blue violet LDs 66 and 67 as much as several nm in the illumination apparatus 14. Such deviations are origins of an individual difference of the endoscope 12 and the illumination apparatus 14. However, the ratio B/G (or B'/G') is equal to the reference ratio according to the image signals B', G' and R of the colors calibrated according to the color calibration table 60 described above. Thus, mode image data generated by use of the B' and G' signals are irrespective of an individual difference of the endoscope 12 or the illumination apparatus 14. Blood vessels as objects of interest can be imaged at a predetermined color balance and definition in the special light mode imaging under an equal condition.

In Fig. 4, the color calibration table 60 is initially created according to the endoscope 12 and the illumination apparatus 14 for use. To this end, the endoscope 12 and the illumination apparatus 14 for use in the imaging are connected to the processing apparatus 13 at first in a step S10. Then a reference chart is imaged by the endoscope 12 with the illumination apparatus 14 in a step S11. The reference chart is a color chart of a surface color of body tissue in a simulated form of spectra of light reflected by body tissue, and has reddish appearance. Details of the reference chart will be described later.

Then the CPU 51 in the processing apparatus 13 creates the color calibration table 60 and writes this to the ROM 57 in a step S12. The CPU 51 obtains conversion data for associating a signal level of an image signal upon imaging of the reference chart with a signal level of a reference image signal for each one of the primary colors, so that the signal level of the image signal of the colors output by the CCD 21 after imaging the reference chart in the step S11 can be equal to the signal level of a reference image signal of the colors. The obtained conversion data is the color calibration table 60. Also, the data of the color calibration table 60 is so determined that a ratio between the image signals of the colors after the calibration is equal to a predetermined reference ratio, or that the ratio B/G of the blue signal to the green signal after the calibration is equal to a reference ratio.

Note that signal levels of image signals of the colors and a reference ratio between the image signals in relation to imaging of a reference chart are predetermined by imaging of the reference chart by use of the reference endoscope 12 and the reference illumination apparatus 14 prepared initially.

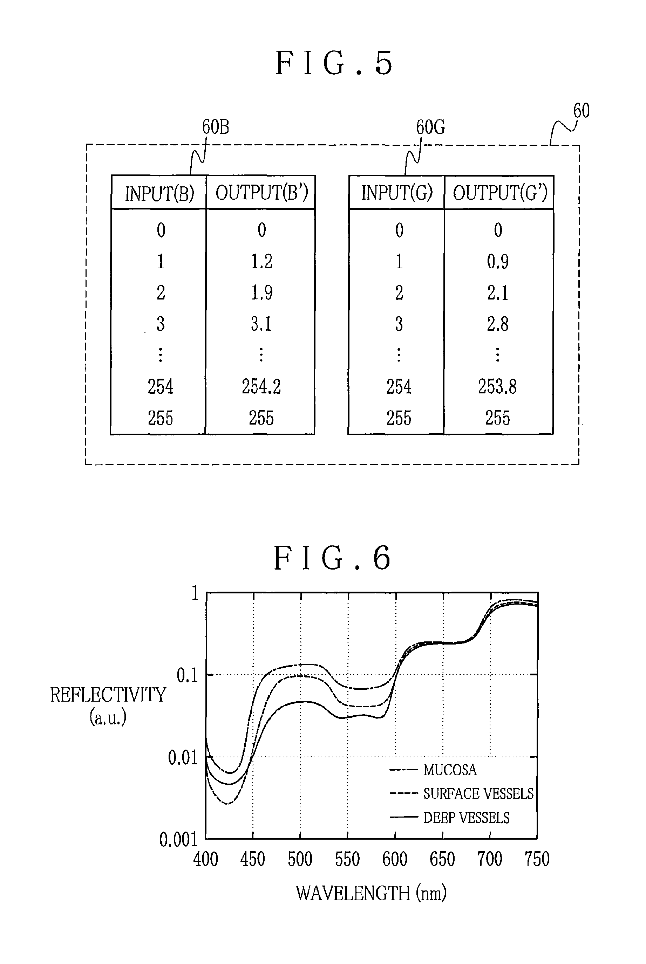

In Fig. 5, the color calibration table 60 includes a blue conversion table 60B for converting the blue signal into a reference value, and a green conversion table 60G for converting the green signal into a reference value.

The blue conversion table 60B is a dataset for calibrating the blue signal input by the CCD 21 to the digital signal processing device 52, and associates a signal level of the blue signal with a signal level of a B' signal after the calibration. The signal level of the blue signal is changed to calibrate the blue signal into the B' signal according to the blue conversion table 60B. Also, the green conversion table 60G is a dataset for calibrating the green signal input by the CCD 21 to the digital signal processing device 52, and associates a signal level of the green signal with a signal level of a G' signal after the calibration. The signal level of the green signal is changed to calibrate the green signal into the G' signal according to the green conversion table 60G.

In the endoscope system 11, a red signal is not used for image data in the special light mode imaging. There is no red conversion table for converting a red signal into a reference value. However, a red signal may be used for image data. It is preferable previously to form a red conversion table by way of the color calibration table 60 in a manner similar to the blue and green conversion tables 60B and 60G.

Data of the blue and green conversion tables 60B and 60G in the color calibration table 60 are determined so that a ratio B/G between the B' and G' signals after the calibration becomes equal to a predetermined value for each of wavelengths, irrespective of an individual difference of the endoscope 12 or the illumination apparatus 14.

Image signals of the colors from the CCD 21 are determined according to spectra of light reflected by body tissue, spectra of light generated by the illumination apparatus 14, combination of spectra of color filters used with the CCD 21 and the like. The spectra of the light and the color filters are changed according an individual difference of the illumination apparatus 14 and the endoscope 12 (CCD 21) with characteristics. If the illumination apparatus 14 or the endoscope 12 is exchanged for use, signal levels of the image signals of the colors are changed according to the individual difference of the illumination apparatus 14 and the endoscope 12.

In Fig. 6, distribution of spectra of light reflected by body tissue is illustrated. There are differences in the spectra between mucosa, surface blood vessels, deep blood vessels (subsurface blood vessels with a large depth or medium depth) and other specific body tissue. However, reflectivity of body tissue for each wavelength range changes in a manner common to any parts of body tissue. Specifically, the reflectivity is lower with blue light of a wavelength of 400-450 nm than with green light and red light of a longer wavelength. The reflectivity is higher with the green light of a wavelength of 450-600 nm than with the blue light, but lower than with the red light of a longer wavelength. In relation to the red light of a wavelength of 600-750 nm, the reflectivity changes in a wavelength of 650-700 nm, but is greatly high. The spectra of reflected light are derived from absorption of hemoglobin.

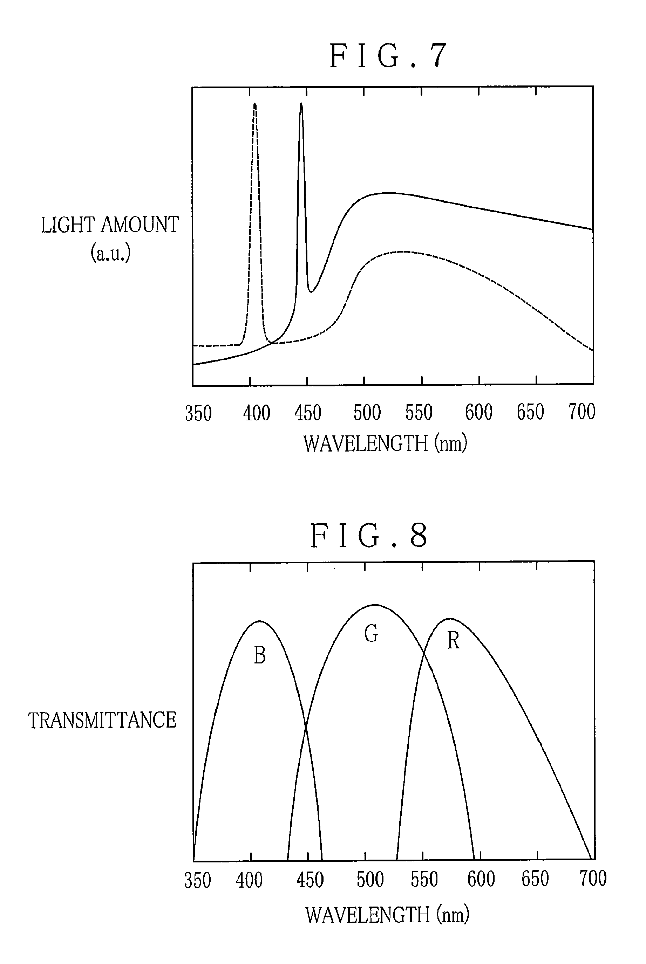

In Fig. 7, light applied to the object is composite light obtained by a combination of white light (solid line) after excitation of blue laser light with a wavelength of 445 nm, and white light (broken line) after excitation of blue violet laser light with a wavelength of 405 nm.

Center wavelengths of the blue laser light and blue violet laser light are 445 nm and 405 nm originally according to the design. However, a deviation as much as plus or minus 2 nm may occur in the center wavelengths. The efficiency in light emission of excitation of the phosphor 43 is influenced by the deviation, to increase or decrease the light emitted by excitation. An individual difference of the illumination apparatus 14 occurs according to the deviation of the center wavelength of laser light even if the illumination apparatus 14 is the same type and has been manufactured equally. Thus, a difference in spectra of light occurs.

In Fig. 8, spectra of color filters of the CCD 21 are illustrated. A blue color filter B absorbs light of a wavelength of 350-450 nm. A green color filter G absorbs light of a wavelength of 450-600 nm. A red color filter R absorbs light of a wavelength of 550-700 nm. The transmission characteristics of the color filters of the primary colors may change with an individual difference due to irregularity in the course of manufacture and changes with time, even with the same type of the CCD 21 manufactured equally. Fine differences occur in signal levels of the image signals according to the characteristic of the CCD 21 even with the same type of the endoscope 12.

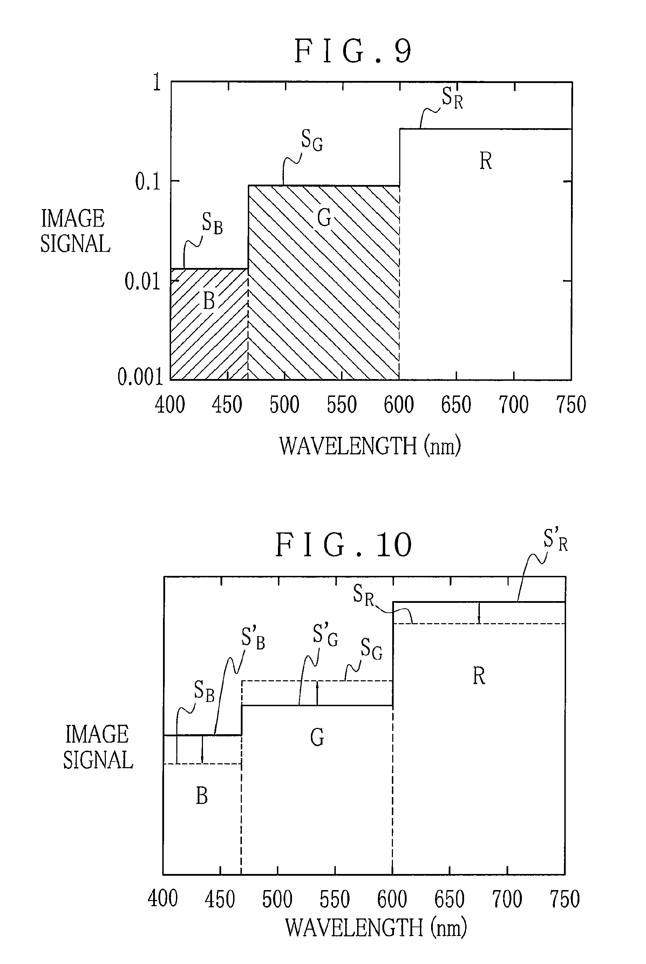

The reference endoscope 12 and the reference illumination apparatus 14 with known characteristics are connected to the processing apparatus 13. When a reference chart is imaged by the endoscope 12, a signal level of an image signal outputted by the CCD 21 is determined according to spectra of light reflected by the reference chart, spectra of reference light, and spectra of a color chart as a reference. As the reference chart is prepared with a surface color of body tissue (to simulate spectra of light reflected by body tissue), the signal level changes similarly to changes in the spectra of the light reflected by the body tissue. For simplification, it is assumed in Fig. 9 that three color signals are different so that their strength increases in the order of a first wavelength range for a blue signal, a second wavelength range for a green signal, and a third wavelength range for a red signal. Signal levels of the color signals are designated by areas SB, SG and SR of hatched portions. The signal levels SB, SG and SR of the blue, green and red signals are determined at a ratio of SB:SG:SR.

A signal level of an image signal, output by the CCD 21 upon imaging of the reference chart by the endoscope 12 and the illumination apparatus 14 connected to the processing apparatus 13, becomes different between the colors in comparison with the imaging with reference apparatuses of the endoscope 12 and the illumination apparatus 14 because of an individual difference of the endoscope 12 and the illumination apparatus 14. For example, the reference chart is imaged by the endoscope 12 and the illumination apparatus 14 for use. See Fig. 10. The signal level S'B of the blue signal is higher than a reference signal level SB due to differences in the source light, color filters and spectra. Similarly, the signal level S'G of the green signal is lower than a reference signal level SG. The signal level S'R of the red signal is higher than a reference signal level SR.

Then the CPU 51 of the processing apparatus 13 calculates data of the blue and green conversion tables 60B and 60G under a condition in which the signal level S'B of the blue signal is equal to the reference signal level SB and the signal level S'G of the green signal is equal to the reference signal level SG. The ratio B/G between the blue and green signals calibrated with the color calibration table 60 is equal to the reference ratio SB/SG.

If it is desired to create a red conversion table as the color calibration table 60, the red conversion table is created under a condition in which a signal level S'R of a red signal is equal to a reference signal level SR.

Owing to the color calibration table 60 determined above, signal levels of the image signal for use in mode image data are equal to reference signal levels even with an individual difference of the endoscope 12 and the illumination apparatus 14. A ratio B/G of the blue signal to the green signal after the calibration is equal to the reference ratio SB/SG. It is possible in the endoscope system 11 to obtain mode image data with constantly determined color balance irrespective of the individual difference of the endoscope 12 or the illumination apparatus 14 for use.

In the endoscope system 11, the digital signal processing device 52 operates for the white balance control at the time of creating normal image data. In principle, the white balance control is based only on a white color as reference. Signal levels of the primary colors are adjusted irrespective of spectra of light reflected by body tissue. There is no maintenance of a ratio between the signal levels of the colors.

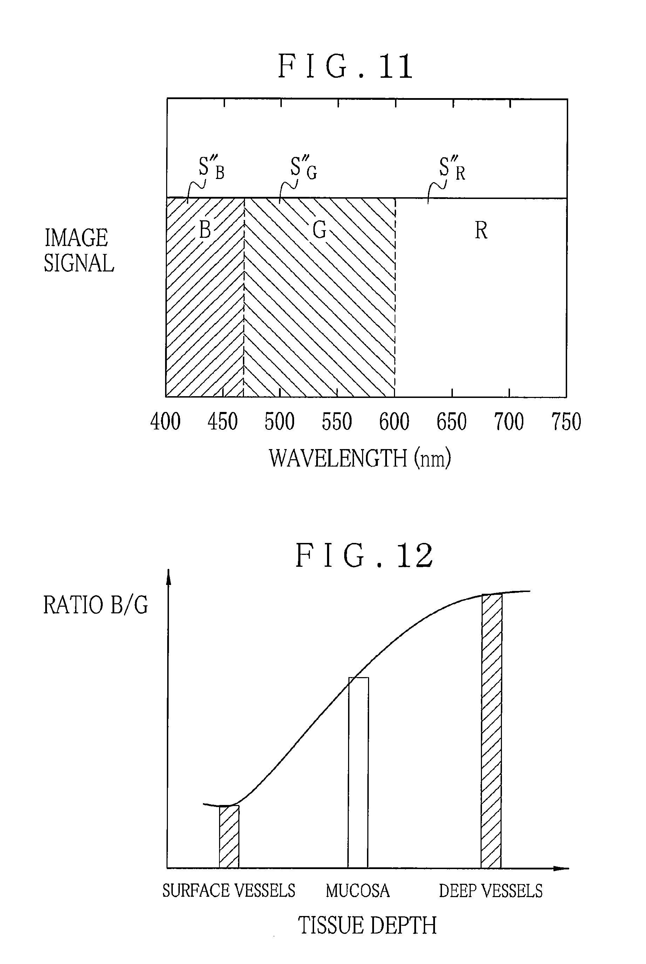

In Fig. 11, the white balance is controlled to obtain white light inclusive of light components of all wavelengths by adjusting signal levels of the colors. The signal levels S"B, S"G and S"R after the white balance control are different from the reference signal levels SB, SG and SR. The ratio B/G between the blue and green signals is different from the reference ratio SB/SG.

An image signal after calibrating a signal level by the white balance control is likely to change a color balance of mode image data with an influence of the individual difference of the endoscope 12 and the illumination apparatus 14 for use. If a combination of the endoscope 12 with the illumination apparatus 14 for use is not favorable, it is likely that visual recognition is low due to low contrast of surface blood vessels and deep blood vessels as a result of an individual difference of the endoscope 12 and the illumination apparatus 14 even in special light mode imaging. However, the signal levels of the image signals of the colors are calibrated according to the color calibration table 60 so as to set the ratio B/G of the blue and green signals equal to the reference ratio SB/SG. It is possible to keep good color balance and definition of surface blood vessels and deep blood vessels irrespective of the individual difference of the endoscope 12 or the illumination apparatus 14.

In the endoscope system 11, the ratio in the signal level between the colors is constant irrespective of an individual difference of the endoscope 12 or the illumination apparatus 14. This feature is preferable specifically for extracting body tissue or a feature image, such as blood vessels.

In Fig. 12, there is a constant relationship between the ratio B/G of the blue and green signals and blood vessels as specific examples of body tissue, such as surface blood vessels and deep blood vessels. In the surface blood vessels, blue light is absorbed remarkably and reflectivity of green light is high, because of a tissue depth of the surface blood vessels under the mucosa and absorption of hemoglobin. Thus, the ratio B/G between the blue and green signals is low because of the low blue signal and high green signal in the surface blood vessels. In the deep blood vessels, green light is absorbed remarkably and reflectivity of blue light is high. Thus, the ratio B/G is high because of the low green signal and high blue signal in the deep blood vessels.

For extracting the surface blood vessels and the deep blood vessels, first and second threshold values are used. The threshold values are previously obtained by experiments, and stored in a memory. If the ratio B/G is equal to or less than the first threshold value, pixels are detected as partial images of the surface blood vessels. If the ratio B/G is equal to or more than the second threshold value, pixels are detected as partial images of the deep blood vessels. If the ratio B/G is more than the first threshold value and less than the second threshold value, pixels are detected as partial images of mucosa.

Accordingly, the endoscope system 11 can easily extract a feature image of an object of interest by image processing, such as surface blood vessels and deep blood vessels, as pixels with a constant value of the ratio B/G between the blue and green signals can be detected. To this end, the digital image processor 53 operates for the image processing to extract vessels and the like. In contrast with this, the image extraction according to the ratio B/G is difficult by the above-described white balance control, because the ratio B/G is different according to an individual difference of the endoscope 12 and the illumination apparatus 14.

In the embodiment, blood vessels as feature image are extracted according to the ratio B/G. However, other information of body tissue can be detected according to the ratio B/G or the image processing, such as oxygen saturation, function information, blood vessel depth and the like. Note that creation of mode image data with enhanced vessels by using the blue signal for the blue and green pixels and using the green signal for the red pixels is an example of signal processing of the feature of the invention.

In the mode image data, the blue signal is used for the blue and green pixels. The green signal is used for the red pixels. Thus, the ratio B/G of the blue signal to the green signal is according to a ratio in the pixel value between the blue and red pixels, or a ratio in the pixel value between the green and red pixels.

In the present embodiment, the special light mode imaging and normal mode imaging can be changed over. The lighting unit 41 has the phosphor 43 for converting the special light into white light. However, the phosphor 43 may not be included in the lighting unit 41. Special light from the blue and blue violet LDs 66 and 67 can be applied to body tissue without use of the white light. In short, an endoscope system of the invention may be specialized for the special light mode imaging without the normal mode imaging.

A second preferred endoscope system is described now. This is in contrast with the above-described embodiment of a full-color type. In the second, the endoscope is a frame sequential type in which a monochromatic image sensor (detection unit) is used. Images of primary colors are created one after another, and are synthesized to obtain a full-color image. According to the feature of the invention, display of the surface blood vessels and the deep blood vessels is suppressed in the frame sequential type. Elements similar to those of the above embodiment are designated with identical reference numerals.

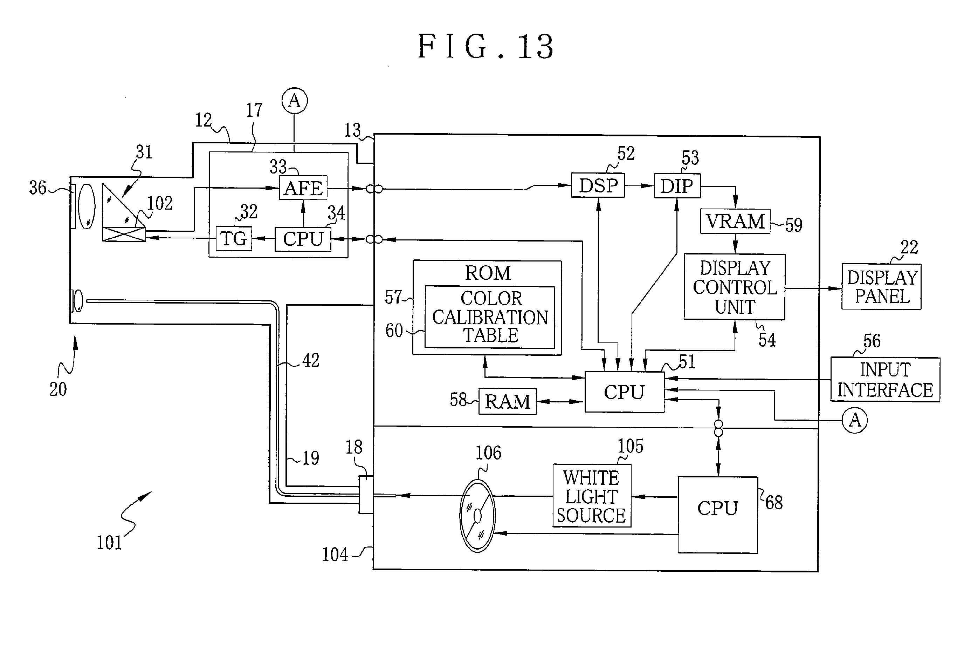

In Fig. 13, an endoscope system 101 is illustrated. A CCD image sensor 102 (detection unit) is incorporated in the endoscope 12. The CCD 102 is monochromatic without a color filter, and operates for imaging of each of the colors by changing over the color of light applied to the body cavity.

An illumination apparatus 104 includes a white light source 105 and a filter wheel 106. Examples of the white light source 105 are a white laser diode, light-emitting diode, xenon lamp and other light source for emitting white light of a broad band. The CPU 68 controls the white light source 105 for a sequence of the light emission and light amount.

The filter wheel 106 is disposed in front of the white light source 105, and converts white light into light of a narrow band of a predetermined wavelength (later to be described), and directs the light to the endoscope 12. The filter wheel 106 has plural regions of filters between which a wavelength of light of a narrow band is different before entry in the endoscope 12. The filter wheel 106 is disposed in a rotatable manner in front of the white light source 105, and rotated in a predetermined sequence by control of the CPU 68. Thus, the wavelengths of narrow bands of light for application to body tissue in a body cavity are changed over sequentially.

Narrow band light, obtained by conversion of source light through the filter wheel 106, is directed to the light guide device 42 by a lens (not shown) or the like, and applied to body tissue of a body cavity through a lighting window with a lens in the head assembly 20 of the endoscope 12.

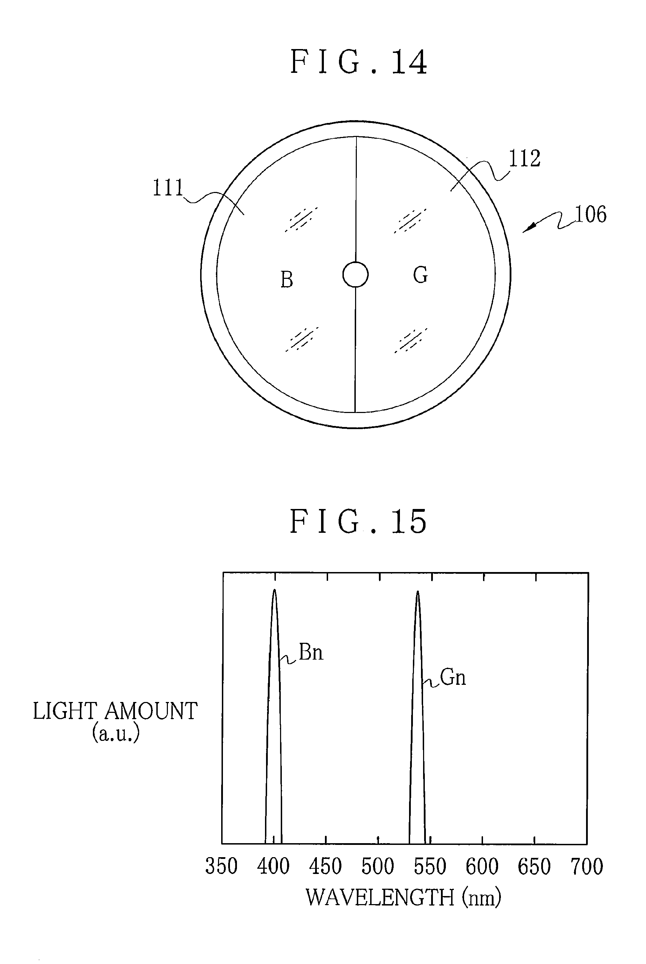

In Fig. 14, the filter wheel 106 includes two filters for transmission of narrow band light, which is light of a very small band width of the wavelength. A blue narrow band filter 111 transmits blue narrow band light Bn. A green narrow band filter 112 transmits green narrow band light Gn. As illustrated in Fig. 15, a wavelength of the blue narrow band light is 415 nm. A wavelength of the green narrow band light is 540 nm. In the present embodiment, the filter wheel 106 has the two regions of the blue and green narrow band filters 111 and 112. However, the filter wheel 106 may have other filters, such as red filter or a region for transmitting light of other colors, or a transmission region for transmitting light totally for all color components, or an opaque region for intercepting light totally for all color components. An angular size of each of the filters may be determined suitably for required time of irradiation specific to each of the colors.

It is preferable to construct a filter wheel by the blue narrow band filter 111, the green narrow band filter 112 and a transmission region (opening or transparency) as three regions. Thus, the operation of imaging can be changed over between the special light mode imaging and the normal mode imaging.

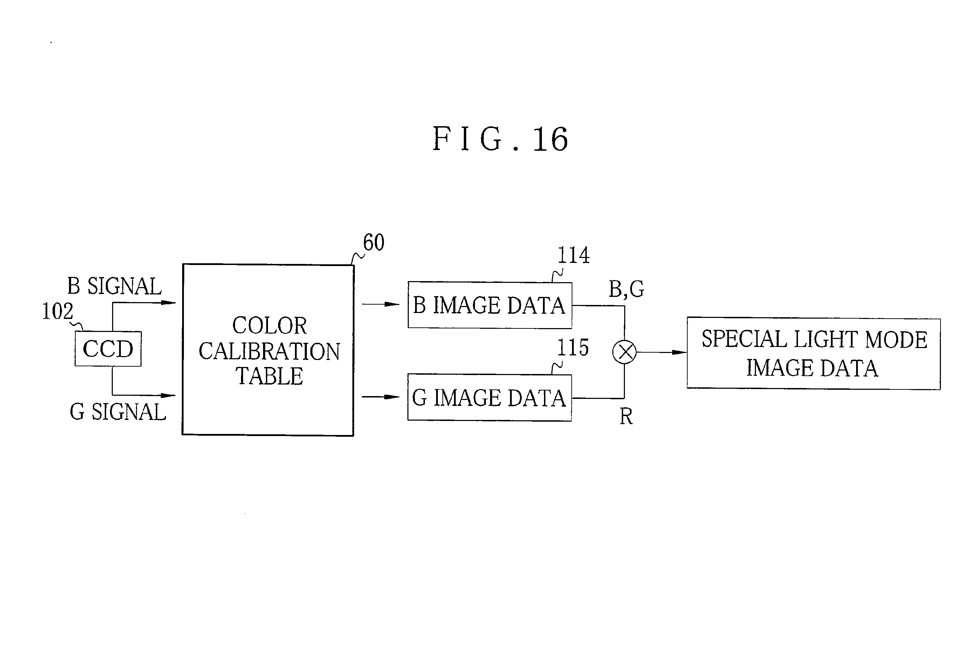

In Fig. 16, blue image data 114 is created by the endoscope system 101 according to a blue image signal (blue signal) after imaging under blue narrow band light Bn, for the purpose of special light mode imaging. The digital signal processing device 52 calibrates the signal level of the blue signal according to the color calibration table 60. Also, green image data 115 is created by the digital signal processing device 52 according to a green image signal (green signal) after imaging under green narrow band light Gn. The digital signal processing device 52 calibrates the signal level of the green signal according to the color calibration table 60. Also, the digital signal processing device 52 creates mode image data by allocating the blue image data 114 to the blue and green pixels and allocating the green image data 115 to the red pixels. Consequently, it is possible to view the contrast of the surface blood vessels and deep blood vessels with predetermined color balance and definition irrespective of an individual difference of the endoscope 12 or the illumination apparatus 104, because the blue and green signals after calibration with the color calibration table 60 are used in the mode image data.

Accordingly, it is possible in the endoscope system 101 to calibrate signal levels of the image signal by use of the color calibration table 60 for creating the blue and green image data 114 and 115. Mode image data can be obtained with constant color balance and definition irrespective of an individual difference of the endoscope 12 or the illumination apparatus 14. The method of creating the color calibration table 60 is the same as the first embodiment.

In the above embodiments, the color calibration table 60 is created by imaging the reference chart before examination by imaging in the endoscope system. However, the invention is not limited.

An example of calibration of an image signal according to an individual difference of the endoscope is described. The endoscope, the processing apparatus and a reference illumination apparatus are used, the reference illumination apparatus being different from that for use. The reference chart is imaged by the endoscope. Thus, a color calibration table is created for calibration of an image signal. It is possible to create the color calibration table for calibrating the endoscope in a discrete manner irrespective of the individual difference of the illumination apparatus. The color calibration table for the endoscope is particularly preferable if an individual difference of the illumination apparatus is extremely small. Also, reference light can be used in place of the reference chart. The reference light is previously adjusted at reflection spectra of a condition of entry of source light on the reference chart. The reference light is directed into the endoscope, to output an image signal. A color calibration table for the endoscope can be created according to the image signal.

Furthermore, an individual difference of an illumination apparatus can be calibrated. The illumination apparatus for use is connected to the processing apparatus. A reference endoscope is used in a discrete manner from the endoscope for use, and images the reference chart with light from the illumination apparatus. Then a color calibration table for calibrating the image signal is created. Thus, the color calibration table can be determined irrespective of an individual difference of the endoscope for calibrating the illumination apparatus discretely. The color calibration table for the illumination apparatus is preferable especially if there is no individual difference in the endoscope, for example, for the endoscope system 101 of the second embodiment with the monochromatic CCD without color filters.

Two color calibration tables may be prepared for calibration of the endoscope and the illumination apparatus in a discrete manner. Furthermore, it is possible to obtain the color calibration table 60 by calculation of the two color calibration tables for a combined use of the endoscope and the illumination apparatus. An example of method for the calculation is described. A memory in the endoscope stores a color calibration table for the endoscope. A memory in the illumination apparatus stores a color calibration table for the illumination apparatus. The endoscope and the illumination apparatus are connected to the processing apparatus 13, in which the CPU 51 determines an optimized form of the color calibration table 60 according to the color calibration tables. Accordingly, it is unnecessary to image a reference chart for the color calibration table 60 shortly before endoscopic imaging. Storing the two color calibration tables for calculating the color calibration table 60 can increase usability of the endoscope system.

In the above embodiments, the color calibration table 60 is created to set the ratio between the image signals of the primary colors equal to the reference ratio. Furthermore, it is possible to create the color calibration table 60 to set a ratio between image signals of only two of the primary colors equal to a reference ratio. For example, the surface blood vessels are imaged with contrast of blue light of a wavelength of 400-450 nm. The deep blood vessels are imaged with contrast of green light of a wavelength of 500-600 nm. As the surface and deep blood vessels are enhanced by image processing, a ratio between the image signals of the two wavelength ranges is very important. Consequently, it is preferable to create the color calibration table 60 by using a reference ratio between the blue signal under a condition of blue light of 400-450 nm and the green signal under a condition of green light of 500-600 nm. To this end, a reference chart can have a surface color of body tissue, or in a form of simulating spectra of light reflected by body tissue at least in the wavelength ranges of 400-450 nm and 500-600 nm.

In the first embodiment, the blue signal is an image signal in the wavelength range of 350-450 nm according to the characteristic of the color filter. The green signal is an image signal in the wavelength range of 450-600 nm. However, the color calibration table 60 can be created to use a reference ratio from a ratio of a partial component of a blue signal according to light of 400-450 nm and a partial component of a green signal according to light of 500-600 nm. In the second embodiment, the blue and green narrow band light Bn and Gn is used. The blue narrow band light Bn is blue in the range of 350-450 nm. The green narrow band light Gn is green in the range of 500-600 nm.

The number of the reference chart for the purpose of creating the color calibration table 60 can be one, or two or more. For example, a plurality of reference charts may be prepared and associated with values of reflectivity of body tissue according to plural tissue depths. The reference charts can be imaged one after another to obtain plural image signals, according to which the color calibration table 60 can be created. Furthermore, one reference chart can include plural regions having high brightness and low brightness. The reference chart can be imaged to obtain plural image signals corresponding to the plural steps of gradation. Note that it is necessary for the reference charts of any of the examples to have a surface color of body tissue, or reflectivity of a simulated form of body tissue.

In the above embodiments, the image signal is calibrated with the color calibration table 60 for special light mode imaging. The white balance control for the image signal is conducted for normal mode imaging. However, it is possible to calibrate the image signal with the color calibration table 60 even for normal mode imaging. For this structure, a red conversion table is previously created in the color calibration table 60 for calibration of red in addition to the blue and green conversion tables 60B and 60G.

In the above embodiment, the ratio B/G after the calibration is set equal to the reference ratio SB/SG. Furthermore, the signal levels S'B and S'G of the colors may be unequal to respectively the reference signal levels SB and SG for the purpose of creating the color calibration table 60. In other words, in the above embodiment, the color calibration table 60 is created to satisfy S'B/S'G = SB/SG by determining that S'B = SB, S'G = SG and S'R = SR. However, data in the color calibration table 60 can be created to satisfy only S'B/S'G = SB/SG without setting the signal levels S'B, S'G and S'R of the colors equal to the reference signal levels SB, SG and SR.

Also, a storage location for storing the color calibration table 60 is not limited to the ROM 57 in the processing apparatus 13 or the like. For example, the color calibration table 60 can be stored in a memory of the endoscope 12 or the illumination apparatus 14. In the endoscope system 11 of a type of full-color imaging, the color calibration table 60 can be preferably stored in the processing apparatus 13 or the endoscope 12. In the endoscope system 101 of a type of a frame sequential imaging, the color calibration table 60 can be preferably stored in the endoscope 12.

In the above embodiments, the endoscope 12 and the illumination apparatus 14 are exchanged with apparatuses of the same types with an individual difference. The individual difference is eliminated by the calibration with the color calibration table 60. However, at least one of the endoscope 12 and the illumination apparatus 14 can be replaced with an apparatus of another type with a structural difference. The calibration with the color calibration table 60 of the invention can be used to compensate for the type difference.

In the first embodiment, the image data is created by allocating the blue signal from the CCD to the blue and green pixels, and allocating the green signal to the red pixels. However, a relationship between an image signal outputted by the CCD 21 and pixels of image data is not limited to this example. In the second embodiment, the mode image data is created by using the blue image data 114 for the blue and green pixels, and using the green image data 115 for the red pixels. However, a relationship between image data of the colors and pixels of image data is not limited to this example.

In the above embodiments, the signal level is calibrated for the digital signal processing device 52 to create the mode image data. However, the digital signal processing device 52 may operate differently. For example, the digital signal processing device 52 may create image data by each color according to image signals from the CCD. The digital image processor 53 can combine the image data of the colors by image processing, so as to create an image with controlled color balance in the manner of the above embodiments.

In the above embodiments, the image sensor is the CCD image sensor. However, CMOS or other image sensor may be used. The number, position and the like of the image sensors can be determined suitably for the purpose.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An electronic endoscope system for imaging body tissue in a tube of a body cavity, comprising:
an illumination means (14, 41, 104) for applying imaging light to said body tissue;
a detection means (12, 21, 102) for imaging said body tissue illuminated with said imaging light, and for outputting a first color image signal (S'B) of a color of a first wavelength range and a second color image signal (S'G) of a color of a second wavelength range;
a calibration means (52, 60) for referring to a reference ratio (SB/SG) predetermined according to a reflectivity of said body tissue in relation to said first and second wavelength ranges, and calibrating said first and second color image signals to set a ratio between said first and second color image signals equal to said reference ratio.

2. An electronic endoscope system as defined in claim 1, further comprising a signal processing means for detecting specific body tissue in said body tissue according to said first and second color image signals.

3. An electronic endoscope system as defined in claim 1, wherein said calibration means carries out calibration when at least one of said illumination means and said detection means is exchanged.

4. An electronic endoscope system as defined in claim 1, wherein said reference ratio is determined according to said first and second color image signals obtained by imaging with a reference illumination means and a reference detection means.

5. An electronic endoscope system as defined in claim 4, wherein said reference ratio is determined by a reference image signal obtained by imaging a reference chart, and said reference chart has a spectrum of reflection of said body tissue.

6. An electronic endoscope system as defined in claim 4, wherein said first wavelength range is equal to or more than 400 nm and equal to or less than 500 nm, and said second wavelength range is equal to or more than 500 nm and equal to or less than 600 nm.

7. An electronic endoscope system as defined in claim 5, further comprising a memory for storing a color calibration table for associating a second pair of said first and second color image signals with a first pair of said first and second color image signals, said second pair being obtained by imaging said reference chart by use of said illumination means for use and said detection means for use, said first pair being obtained by imaging said reference chart by use of a reference illumination means and a reference detection means;
wherein said calibration means carries out calibration according to said color calibration table.

8. An electronic endoscope system as defined in claim 4, further comprising a display control unit for creating a display image by use of said first and second color image signals.

9. An electronic endoscope system as defined in claim 8, wherein said display control unit creates a special image for said display image by allocating a signal level of said first color image signal to blue and green pixels, and allocating a signal level of said second color image signal to red pixels.

10. An electronic endoscope system as defined in claim 2, wherein said signal processing means evaluates said ratio of said first and second color image signals by pixels, and extracts said specific body tissue from an image of said body tissue according to evaluation of said ratio.

11. An electronic endoscope system as defined in claim 1, wherein said detection means includes a color image sensor;
said illumination means includes:
a blue laser for generating a blue laser beam;
a blue violet laser for generating a blue violet laser beam;
phosphor, disposed at a distal end of an endoscope having said detection means, for generating fluorescent light from green to yellow upon emission of said blue laser beam and said blue violet laser beam, wherein white light is generated in combination of said fluorescent light and blue light upon passage through said phosphor, and becomes said imaging light to said body tissue.

12. An electronic endoscope system as defined in claim 3, wherein said imaging light is white light, and said detection means includes a color image sensor.

13. An electronic endoscope system as defined in claim 3, wherein said imaging light contains blue narrow band light and green narrow band light applied to said body tissue sequentially, said detection means includes a monochromatic image sensor for outputting said first and second color image signals serially.

14. A calibration method of calibrating a first electronic endoscope system by use of a reference electronic endoscope system, wherein each of said reference endoscope system and said first endoscope system includes an illumination means (14, 41, 104) for applying imaging light to body tissue in a tube of a body cavity, and a detection means (12, 21, 102) for imaging said body tissue illuminated with said imaging light, and for outputting a first color image signal (S'B) of a color of a first wavelength range and a second color image signal (S'G) of a color of a second wavelength range, said calibration method comprising steps of:
imaging a reference chart by use of said reference endoscope system to obtain a first pair of said first and second color image signals, said reference chart having a color pattern according to a surface color of said body tissue;
determining a reference ratio (SB/SG) between said first and second color image signals of said first pair;
imaging said reference chart by use of said first endoscope system to obtain a second pair of said first and second color image signals;
determining a ratio between said first and second color image signals of said second pair;
calibrating said second pair of said first and second color image signals by setting said ratio equal to said reference ratio, to control a color balance of an image output by said first endoscope system.

15. A calibration method as defined in claim 14, wherein said illumination means is used commonly between said reference endoscope system and said first endoscope system.

16. A calibration method as defined in claim 14, wherein said calibrating step is carried out when at least one of said illumination means and said detection means in said first endoscope system is exchanged.
